# EUROPEAN PATENT APPLICATION

(11) **EP 4 183 889 A1**
(43) Date of publication of application: **24.05.2023**
(21) Application number: 21383048.2
(22) Date of filing: 22.11.2021
(51) Int. Cl.: C12Q 1/6888

(54) **METHOD FOR THE IDENTIFICATION AND RAPID DETECTION OF BLUEFIN TUNA**

(71) Applicant: Consejo Superior De Investigaciones Científicas (CSIC), 28006 Madrid (ES); INL - International Iberian Nanotechnology Laboratory, 4715-330 Braga (PT)
(72) Inventor: Muñoz Colmenero, Ana Marta, 28006 Madrid (ES); González Sotelo, Carmen, 28006 Madrid (ES); Prado, Marta, 4715-330 Braga (PT); Garrido-Maestu, Alejandro, 4715-330 Braga (PT)
(74) Representative: Neves, Ana

(57) **Abstract**

The present invention discloses a method for fast and unambiguous identification of bluefin tuna *(Thunnus thynnus).* The identification is based on isothermal amplification and specific primers and probes, able to distinguish the species *T. thynnus* from other related species. This is an important improvement in detection of fraud in bluefin tuna commercialization. The method disclosed therein is a fast and portable method which do not require laboratory facilities and may be performed by non-specialized personnel, which allows control of the species reliable and fast in any place by any person.

## Description

### FIELD OF THE INVENTION

The present invention relates to a method of identification of the *Thunnus thynnus* species by isothermal amplification. The present invention belongs to the field of food, more specifically to the control of food species for consumption.

### BACKGROUND OF THE INVENTION

Bluefin tuna, "Atún rojo" or Atlantic bluefin tuna *(Thunnus thynnus)* is a species of tuna belonging to the Scombridae family. Bluefin tuna is native to both shores of the Atlantic Ocean and the Mediterranean Sea.

Bluefin tuna has large amounts of omega 3 fatty acids, vitamins B (2, 3, 6, 9 and 12) and E and minerals such as selenium and magnesium. As other marine organisms, this species is associated with the prevention of cardiovascular diseases (hypercholesterolemia, hypertriglyceridemia, hypertension, diabetes, obesity, angina pectoris, heart attack, thrombosis, cerebral stroke or atherosclerosis) and brain diseases. It is important to note that it should not be consumed in excess since, being a very long-lived fish, it accumulates mercury.

Given its gastronomic interest and healthy properties, *Thunnus thynnus* species is highly appreciated by consumers.

However, it is common finding cheaper fish mislabeled as bluefin tuna without advising consumers. This fraud is very common unfortunately, and the responsibility would be of the fish provider or the company selling the product.

To facilitate that any small company may identify this fraud, it is necessary a quick and easy procedure to specifically identify *T. thynnus* species in commercial fishery products from other nearby species. This would allow to verify that the food being marketed corresponds to the tagged species.

Document CN111748609A discloses primers and methods for identifying fish-derived components (i.e., *T. thynnus).* Mitochondrial DNA was used as the target sequence to design the specific primers. The reaction for detection is a real-time fluorescence multiplex PCR in order to detect bluefin tuna, silver cod and school fish. The SYBR green fluorescent quantitative PCR melting curve analysis method is used to develop a multiplex system that can simultaneously detect bluefin tuna, silver cod, and schoolfish species. This method takes at least 50 minutes to be performed. In addition, a real time PCR requires formed personnel to use a complex expensive and non-portable apparatus.

Document CN102732630A discloses a method to identify the origin of *Thunnus obesus, Thunnus alalunga* and *Thunnus thynnus* sashimi which consists of extracting the DNA from a tuna sashimi, using it as a template and F1/F2 primers, carrying out a PCR amplification of the gene fragments of the mitochondrial cytochrome (cytb), electrophoresis detection of the amplified fragments, enzymatic digestion of the PCR products with the restriction enzymes Fokl and Stul after purified, and identify the origin of the sashimi. The method described in this document requires at least a thermocycler (minimum 1 hour), gel electrophoresis (which requires also specific equipment and use of a cabinet to avoid toxic mist), restriction enzymes... which results in time and resources.

Document ES 2159234 A1 discloses a method for the identification of bluefin tuna *(Thunnus thynnus),* bigeye *(Thunnus obesus)* and skipjack *(Katsuwonus pelamis)* species in canned tuna, and yellowfin tuna *(Thunnus albacares)* in canned tuna and canned yellowfin tuna. The procedure is characterized by amplifying a fragment of 187 bp (bdr) of the cytochrome b gene located in the mitochondrial DNA, by the sequential use of a set of restriction endonucleases and the analysis of the resulting fragments that allow differentiating the red (bluefin), bigeye and skipjack species from other species that can be used as substitutes in canned tuna.

However, none of these documents provides a rapid identification methodology for *T. thynnus,* independent of sophisticated equipment or specific training. In all of the procedures disclosed in the prior art at least one hour (usually more) is necessary to complete the whole procedure, and it is also necessary to have molecular biology knowledge to perform the techniques and handle the necessary equipment. Furthermore, none of these documents allow identification of this species "on-site", making the analysis dependent on an equipped laboratory. Finally, none provides a user-friendly methodology which allows the visualization of the final result in a single view in a few minutes.

Therefore, is necessary a new method for identification and rapid detection of the species *Thunnus thynnus* independent from a laboratory facility.

### DESCRIPTION OF THE INVENTION

### Brief description of the invention

The present invention discloses a process for identification of bluefish tuna *(Thunnus thynnus)* from a fish sample comprising:
a) DNA isolation from the sample;
b) adding to the isolated DNA of step a) both forward primer of sequence SEQ ID NO: 1 and a reverse primer of SEQ ID NO:2;
c) adding an enzyme;
d) incubating the mixture of step c) between 10 and 30 minutes at 25 - 42 °C; and
e) visualization of the result,
wherein the enzyme is selected from a recombinase (RPA), a DNA helicase (HAD) or a strand-displacement enzyme (SDA).

### Description of the figures

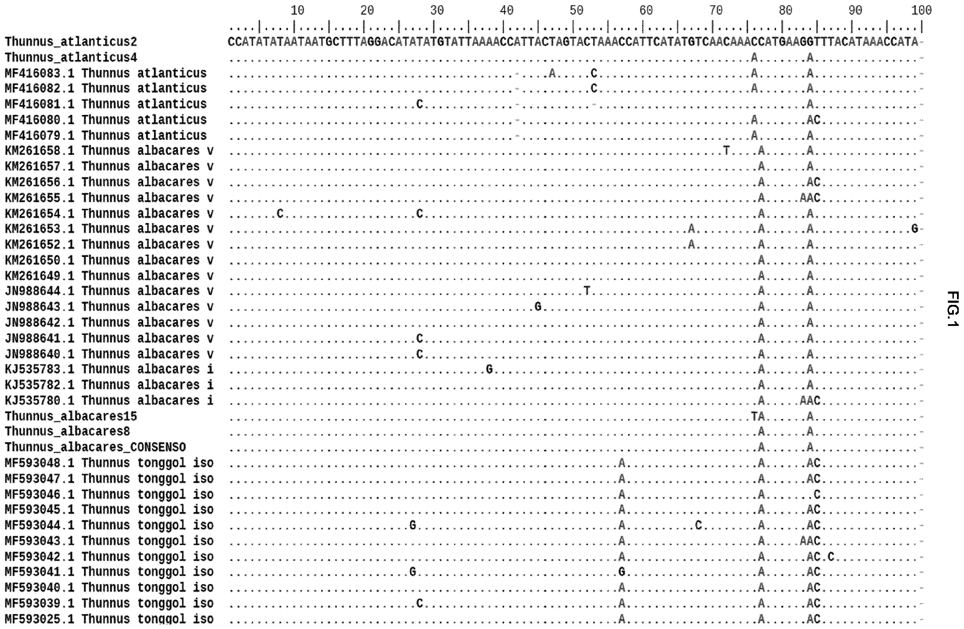

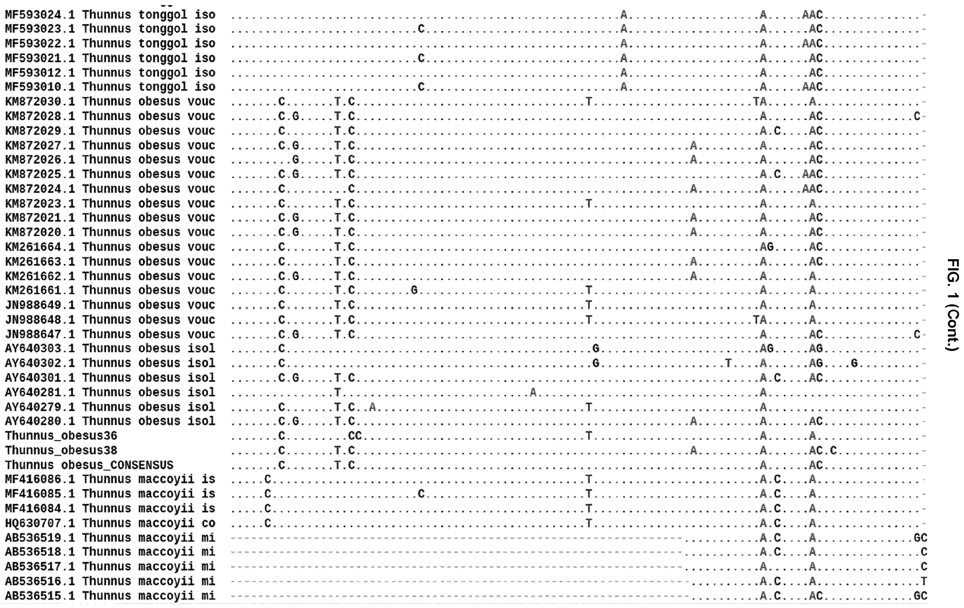

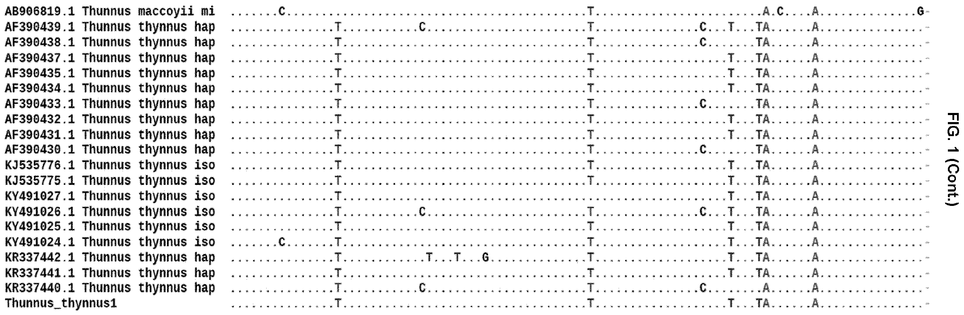

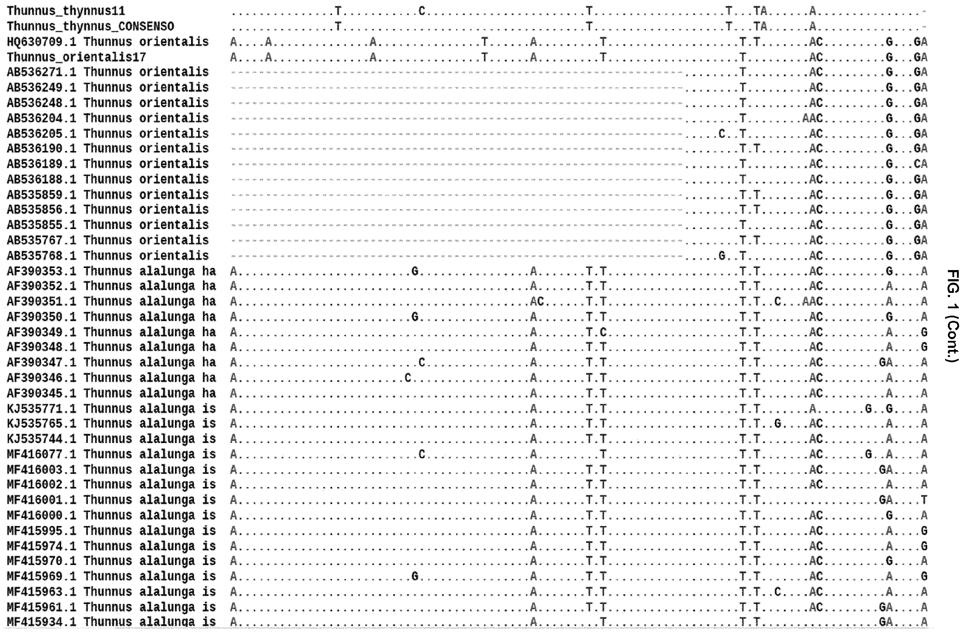

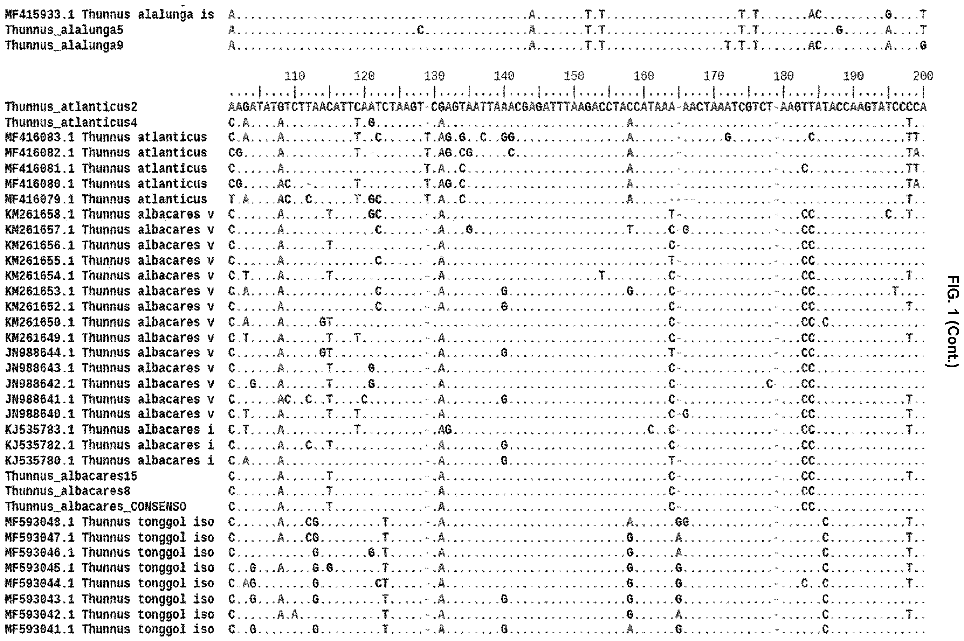

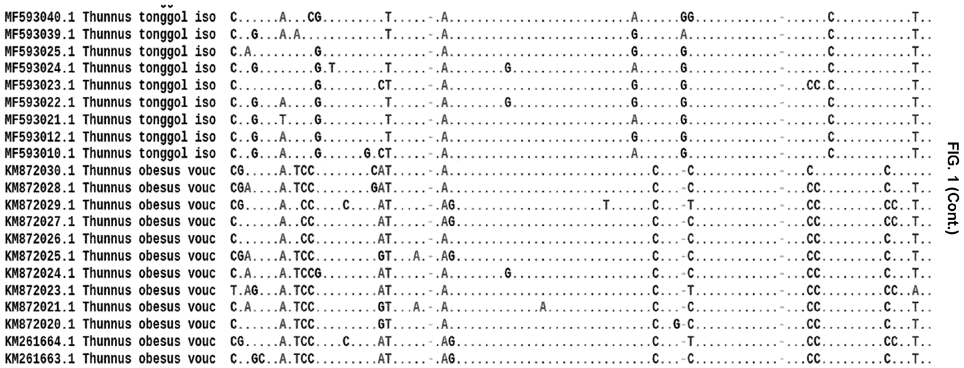

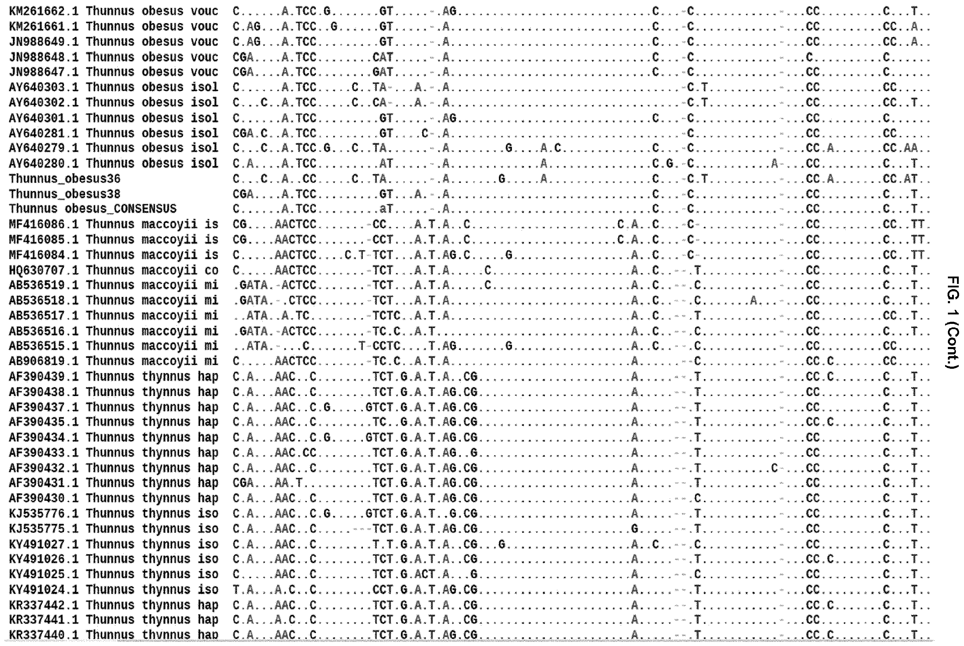

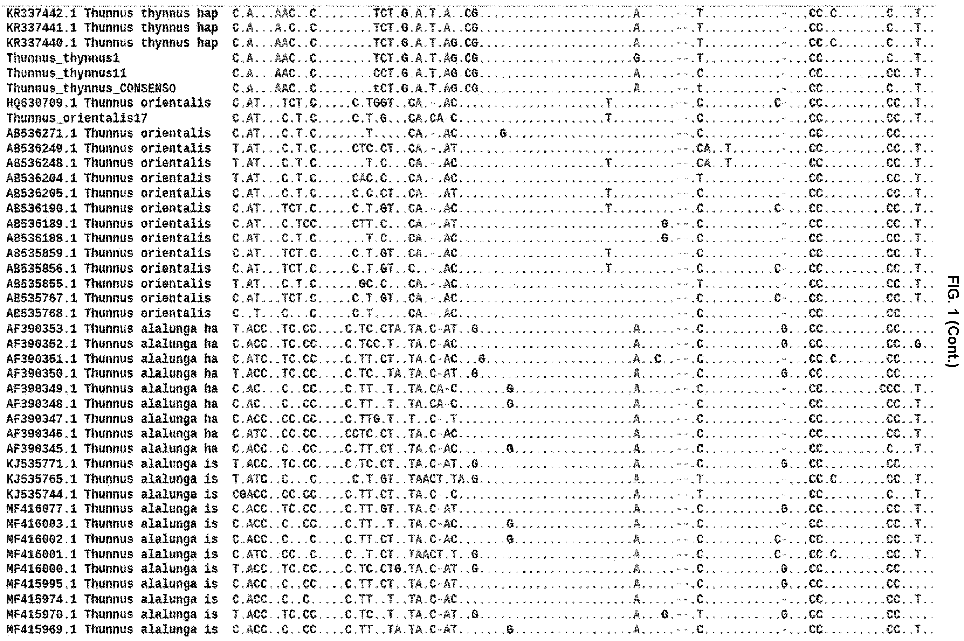

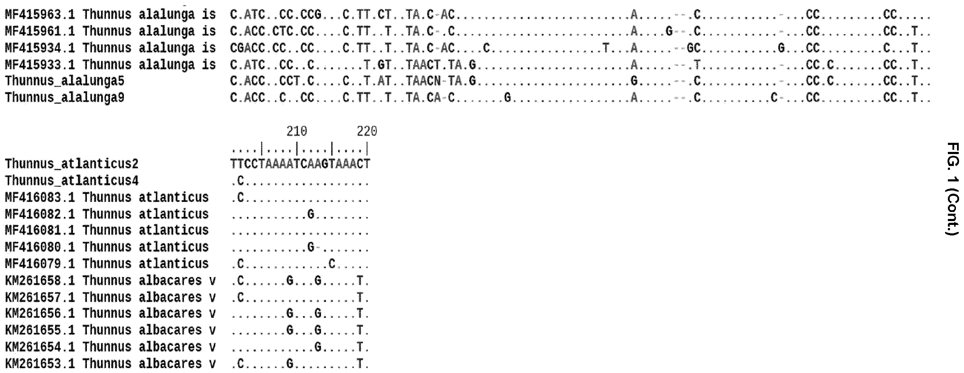

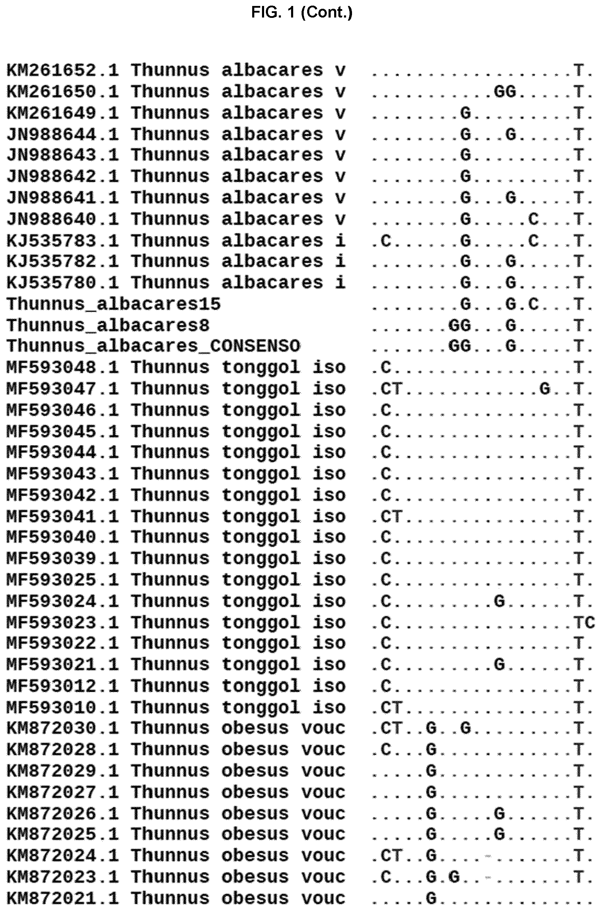

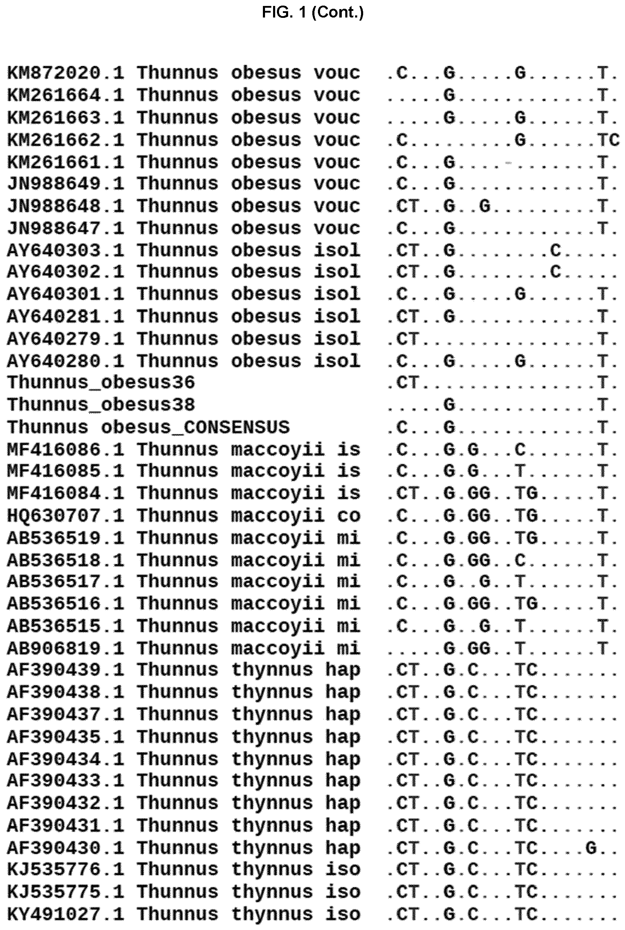

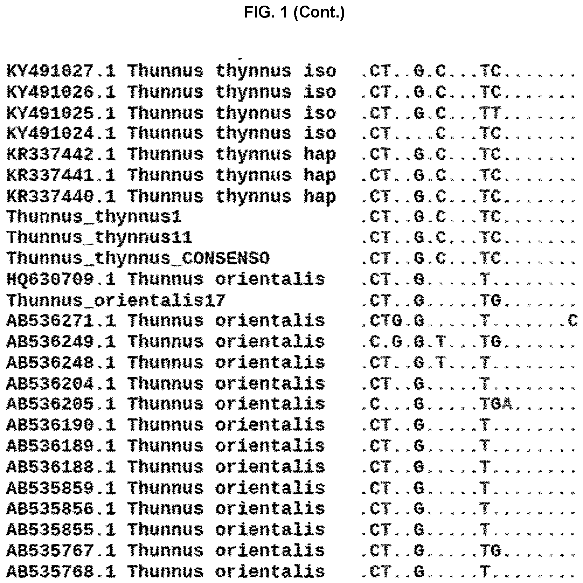

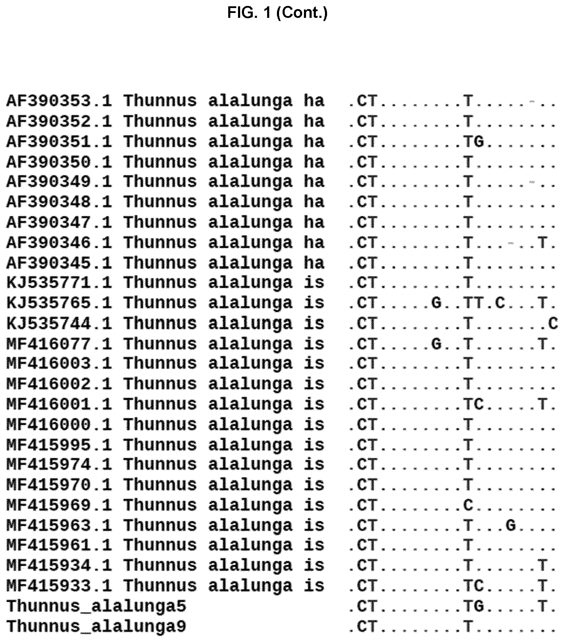

**Figure 1****.** Alignment of CR with all sequences cut and selected for the primers and probes.

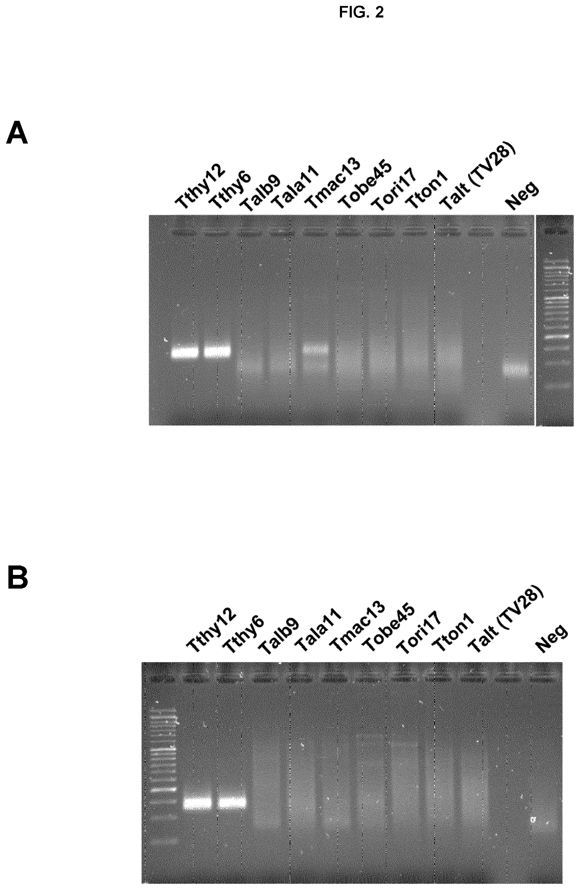

**Figure 2****.** Agarose gel showing RPA basic amplification with the primer sets F1-R3 (A) and F2-R3 (B). The samples assessed were: Tthy15 *(T. thynnus),* Tthy6 *(T. thynnus),* Talb9 *(T. albacares),* Tala11 *(T. alalunga),* Tmac13 *(T. maccoyii*), Tobe45 *(T. obesus),* Tori17 *(T. orientalis),* Tton17 *(T. tonggol*), Tatl18 *(T. atlanticus)* and NEG (Negative control without DNA).

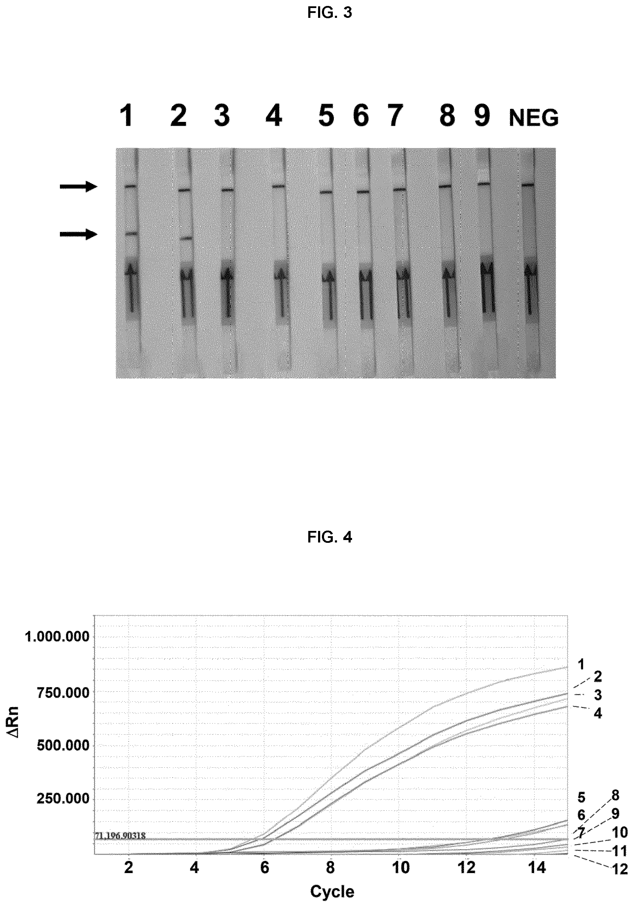

**Figure 3****.** LFA results with the F2-R3 primer set and P2 nfo probe for *Thunnus* species. The samples assessed were: 1: Tthy12 *(T. thynnus),* 2: Tthy6 *(T. thynnus),* 3: Talb9 *(T. albacares),* 4: Tala11 *(T. alalunga),* 5: Tmac13 *(T. maccoyii*), 6: Tobe45 *(T. obesus),* 7: Tori17 (*T*. *orientalis),* 8: Tton1 *(T. tonggol*), 9: TatlTV28 *(T. atlanticus)* and NEG: Negative control (no DNA).
**Figure 4****.** Fluorescence results with the F2-R3 primer set and exo probe for *Thunnus* species. The samples assessed were: 1: BAL3 (*T*. *thynnus),* 2: BAL1 (*T*. *thynnus),* 3: GAD1 (*T*. *thynnus),* 4: TTHY15 (*T*. *thynnus),* 5: NEG (Negative control (no DNA), 6: TTON17 (*T*. *tonggol*), 7: TATL18 (*T*. *atlanticus),* 8: TORI27 (*T*. *orientalis),* 9: TALB9 (*T*. *albacares),* 10: TMAC13 (*T*. *maccoyii*), 11: TOBE45 (*T*. *obesus),* TALA11 (*T*. *alalunga).*

### Detailed description of the invention

In order to resolve the aforementioned problems, the present invention discloses a process for unambiguous identification *T*. *thynnus.* This process is a fast DNA-based method. The process comprises specific amplification of *T*. *thynnus* (without amplifying or detecting any other species of the *Thunnus* genus). This is a breakthrough in species identification, since allow distinguishing *T*. *thynnus* from the main substitutes in marketed products, such as *T*. *atlanticus, T*. *albacares, T*. *tonggol, T*. *obesus, T*. *maccoyii, T*. *orientalis* and *T*. *alalunga.*

In a first embodiment, an object of the present invention is a method to identify the species *Thunnus thynnus* from a fish sample comprising:
a) DNA isolation from the sample;
b) adding to the isolated DNA of step a) the DNA primers of sequence SEQ ID NO:1 and SEQ ID NO:2;
c) adding an enzyme;
d) incubating the mixture of step c) between 10 and 30 minutes at 25 - 45 °C; and
e) visualization of the result,
wherein the enzyme is selected from a recombinase (RPA), a DNA helicase (HAD) or a strand-displacement enzyme (SDA).

The components of the reaction may be added in any order. Preferably the DNA is added the last, in order to ensure that the reaction does not start in advance and avoid any contamination or unspecific amplification, likewise any amplification reaction. This method is fast (30 minutes or less after the DNA has been isolated) and simple, therefore any small company or could perform the method in its facilities or elsewhere as long as an incubator and a visualizing system is present.

In step a), the DNA is isolated from the fish sample. A fish sample is any tissue of any fish. In a preferred embodiment, the fish sample is selected from muscle tissue. The sample may be fresh, frozen or conserved in oil or canned. The DNA isolation may be performed by any method known by a person skilled in the art.

The forward and reverse primers in step b) are defined respectively by SEQ ID NO:1 and SEQ ID NO:2. These primers have been designed to specifically detect *T. thynnus* without detecting any other related species. In an embodiment, when the enzyme is RPA nfo, each primer is labelled in 5' position. In a more preferred embodiment, only the reverse primer (SEQ ID NO:2) is labeled. Examples of labels are biotin, 6-FAM, TET, VIC, HEX, NED, PET among others. In a preferred more embodiment, the label is biotin.

The concentration of each primer and DNA usually depends on the polymerase manufacturer. In a preferred embodiment, each primer is in a concentration of 100 nM - 10 µM, preferably 300 nM - 5 µM and more preferably, 300 nM - 1 µM. The amount of isolated DNA in the reaction is between 10-50 ng/µL, preferably 25 ng/µL.

The enzyme responsible for the amplification is added to the mixture in step c). This enzyme is selected from the group consisting of a recombinase (RPA), a DNA helicase (HAD) or a strand-displacement enzyme (SDA). These enzymes allow, respectively, recombinase polymerase amplification (RPA), helicase-dependent amplification (HAD) or strand-displacement amplification (SDA). In a preferred embodiment, the amplification is carried out by a recombinase (RPA). In a more preferred embodiment, the RPA is selected from nfo RPA, exo RPA or fpg RPA. In an even more preferred embodiment, the enzyme is nfo RPA.

When a nfo RPA, exo RPA or fpg RPA enzyme is used, a specific probe is also added. The probe for nfo RPA has been specifically designed for *T. thynnus* and corresponds to SEQ ID NO:3. In an embodiment, the probe is labeled with a flurorophore in 5' position and has a blocker in 3'. Preferably the fluorophore in 5' is FAM and the blocker in 3' is selected from a C3-space, a phosphate, a biotin-TEG or an amine. More preferably, the probe is labeled with FAM at 5' and a C3-space at 3'. In an embodiment, the sequence of the probe comprises an abasic/AP site between positions 30 and 35 (from 5' of SEQ ID NO:3). In a preferred embodiment, the abasic site is selected from thetrahydrofurane (THF), dSpacer (a derivative of the THF) or a dR group (deoxyribose of the abasic site via a C-O-C linked). In a more preferred embodiment, the abasic site is THF. In an even more preferred embodiment, the THF is located at position 33 of SEQ ID NO:3 (A→THF). In the most preferred embodiment, the probe when nfo RPA is used is:
5'-FAM-TCATCTTGAATTCAGGCGATTAAACGAGATTT-THF-AGACCTAACATAAAT-[C3-Spacer]-3' .

The probe for the exo RPA has been specifically designed for *T. thynnus* and corresponds to SEQ ID NO:4. This probe is labeled with a fluorophore inside the sequence, has a blocker in 3' and a quencher. Preferably the fluorophore is FAM and the blocker in 3' is selected from a C3-space, a phosphate, a biotin-TEG or an amine. More preferably, the probe is labeled with FAM and a C3-space at 3'. In a preferred embodiment, the FAM is located between positions 25 and 35 (from 5' of SEQ ID NO:4). In a more preferred embodiment, FAM is located at position 31 from 5' of SEQ ID NO:4 (T→FAM). The quencher could be selected from any quencher compatible with the fluorophore. When the fluorophore is FAM, the quencher may be selected for example BHQ1 or TAMRA, among others. In a preferred embodiment, the quencher for FAM is BHQ1, located between positions 30 and 40 (from 5' of SEQ ID NO:4). In a more preferred embodiment, BHQ1 is located at position 36 from 5' of SEQ ID NO:4 (T→BHQ1). The nucleotides to which the fluorophore and the quencher substitute are both timines in the original sequence. The probe also comprises an abasic/AP site. In a preferred embodiment, the abasic site is selected from thetrahydrofurane (THF), dSpacer or a dR group. In a more preferred embodiment, the abasic site is THF. The THF must be located between the fluorophore and the quencher. In a preferred embodiment, the THF is located between positions 25 and 35 (from 5' of SEQ ID NO:4). In an even more preferred embodiment, the THF is located at position 33 of SEQ ID NO:4 (A→THF). The exo RPA does not require labeled primers SEQ ID NO:1 and SEQ ID NO:2. In the most preferred embodiment, the probe when exo RPA is used is:
5'-TACATAAACCATACAAATAAACCTCAACAT-FAM-C-THF-TC-BHQ1-TGAATTCAGGCG-[C3-Spacer]-3'.

The enzyme is added to the mixture in a reaction buffer. This buffer is supplied by the enzyme's manufacturer. For example, this buffer may comprise polyethylene glycol (PEG) 35 K 5%, dithiothreitol (DTT) 2 mM, phosphocreatine 50 nM, creatine kinase 100 ng/µL, Adenosine triphosphate (ATP) 3 mM, Tris(hydromethyl)aminomethane pH 7.9 50 mM, potassium acetate 100 mM 200 µM of each deoxynucleotide triphosphate (A, T, C and G) and magnesium acetate 10 - 15 mM.

When the reaction is a RPA, magnesium acetate is added to the reaction mixture to start the reaction. Distillated water may be also added.

The mixture is incubated in step d) for 10 - 30 minutes at 25-42 °C. In a preferred embodiment, the incubation is at 37 - 42 °C. In a more preferred embodiment, the incubation is for 15 minutes at 38 °C.

Finally, the result of the reaction is visualized in step e). Any independent DNA detection method may be used. In a preferred embodiment, the preferred detection method is selected from real-time fluorescent detection, gel electrophoresis, lateral flow strip detection, flocculation assay detection, electrochemical detection, chemiluminescent detection, silicon micro-ring resonator (SMR)-based photonic detection and surface-enhanced Raman scattering (SERS) detection. In a more preferred embodiment, the detection method is lateral flow strip detection when the enzyme is RPA nfo, RPA fpg or SDA, as it is independent from laboratory facilities which may be used anywhere by a non-trained person.

The method of the invention relies on the combination of the specific sequence of the primers and probe (when nfo RPA, exo RPA or fpg RPA is used), together with the isotherm amplification by RPA (incubation at a fixed temperature). The sequences SEQ ID NO:1 and SEQ ID NO:2 corresponding to the primers and the sequences of the probes (SEQ ID NO:3 for nfo RPA and SEQ ID NO: 4 for exo RPA) are essential to unambiguous identify the species *T. thynnus.* These molecules have been designed in order to detect a genomic fragment with inter-species variability which avoid false positives and specific identification. Despite the documents already published in this sense, this is the first-time unambiguous identification of *T. thynnus* is performed in a simple and fast method. The sequences of the primers are also compatible with other known techniques such as PCR.

Recombinase polymerase amplification is performed a single tube and is an isothermal procedure. This method is an alternative to the polymerase chain reaction (PCR) techniques. However, the method of the invention requires simpler equipment than PCR (for example, it does not require a thermal cycler). The equipment necessary to carry out the method of the invention is simple, easy to use and moveable to any location, which is of high importance to the user, since he/she is not bounded to a specific laboratory and may perform the identification directly in factories, restaurants, production sites, etc.

To date, there are no methods of identification and detection of this species which do not depend on facilities prepared or equipped with large devices, or which require high trained personnel in real-time PCR or next generation sequencing (NGS). The method of the invention facilitates the identification of *T. thynnus* this species of high economic value for its commercialization, where it has been verified that there is a constant and high fraud. Due to the short incubation and detection times, the method after the DNA has been extracted, may be conducted in less than 30 minutes, far beyond to a PCR.

### EXAMPLES

### Example 1. Design of the primers for amplification

A search was carried out in databases [GenBank, Aquagene, (June 2019 - December 2019) and the database of the food biochemistry group of the IIM-CSIC], to analyze the genetic variability present in different genes in order to select the best candidates to design the sequences (primers and probes) for the isothermal amplification system. Additionally, for some genes new sequences were obtained from reference organisms available in the laboratory.

The nuclear genes studied were:
- Rhodopsin: 86 sequences were reviewed with sequences from the eight species of the *Thunnus* genus. The common fragment among the sequences were short and the nucleotide variability was practically nil, being impossible use this fragment to differentiate the species.
- Calmodulin A: Only two sequences were found for this gene, for *T. thynnus* (AB291547.1) and for *T. orientalis* (LC183900.1). Without more sequences was impossible to design a species-specific system to identify *T. thynnus* and differentiate it from the rest *Thunnus* species.
- Titin like protein (TMO-4c4): only three sequences were found *(T. tonggol* DQ388107.1, *T. obesus* DQ388106.1 and *T. alalunga* DQ388108.1), only one sequence for each, and none for the target species. Alignment was around 380 bp with only 2 SNPs, so it does not have enough variability to differentiate between species.
- 18S rRNA: Only five sequences were found in GenBank, four for *T. alalunga* (KT186636.1-KT186639.1) and one for *T. orientalis* (AY695887.1), so there was not enough information or sequence available for the species of interest. Furthermore, the sequences available did not align correctly and that could be because they were of different fragments of this gene or errors in the database.
- 5S rRNA: 36 sequences were downloaded, but only for four species *(T. albacares, T. obesus, T. alalunga, T. orientalis).* For the rest, no available sequences were found. The aligned fragment was about 300 base pairs and showed some SNPs between individuals, although they only allowed to differentiate two of the four species present, so the variability was not enough.
- Myosin heavy chain: One sequence was downloaded from GenBank for *T. albacares* and 3 sequences from Aquagene *(T. albacares, T. alalunga* and *T. thynnus).* The alignment presented very low variability with a few SNPs and only was possible differentiate *T. alalunga* to the rest. Therefore, there was not enough variability to differentiate Bluefin tuna.
- ITS1: 65 sequences were downloaded from GenBank, covering the eight species included in the genus *Thunnus.* The variability present in this fragment was studied for these species and the *T. thynnus* identification options. Various SNPs were found that could differentiate some species, but no possibilities were found to differentiate Bluefin tuna due to the high similarity with *T. orientalis.* New sequences were amplified for this region in the inventor's laboratory to see if, by increasing the sequences studied, it could be found new possibilities in this gene, but no new SNPs were obtained.

Mitochondrial genes were also analyzed:
- Cytocrome b (Cyt b): This gene is one of the most widely used for the differentiation of tuna of *Thunnus* genus through the use of general primers for this genus and subsequent sequencing to identify SNPs, diagnoses of each species and/or comparison by BLAST with databases or FINS. The SNPs present in this gene were studied from sequences downloaded from GenBank as well as from the inventor's laboratory sequences (amplified with the primers of Kocher et a/. 1989 (PNAS 86(16); 6196-6200), for the eight species of the genus *Thunnus.* Despite the diagnostic SNPs present in this gene, there was not found a method to design a specific system only for *T. thynnus* due to (1) high similarity among the species and (2) the distance between the SNPs present did not meet the requirements needed to apply the isothermal amplification. For these reasons, this gene was not considered suitable for the design of the primers and probe. Furthermore, with the variation of the fragment it seemed impossible to find diagnostic differences between the species of interest *T. thynnus* and the species *T. macoyii.*
- Control region (CR): To analyze the variability available in this fragment, sequences were downloaded from GenBank and aligned with sequences that had been obtained by the inventors. In total, 144 DNA sequences were reviewed. After studying this alignment, several SNPs with diagnostic potential were detected as well as two hypervariable regions with great potential for the design of specific primers. Table 1 shows the SNPs present in the alignment for all *Thunnus* species. The numbers correspond to the number of positions in the alignment.

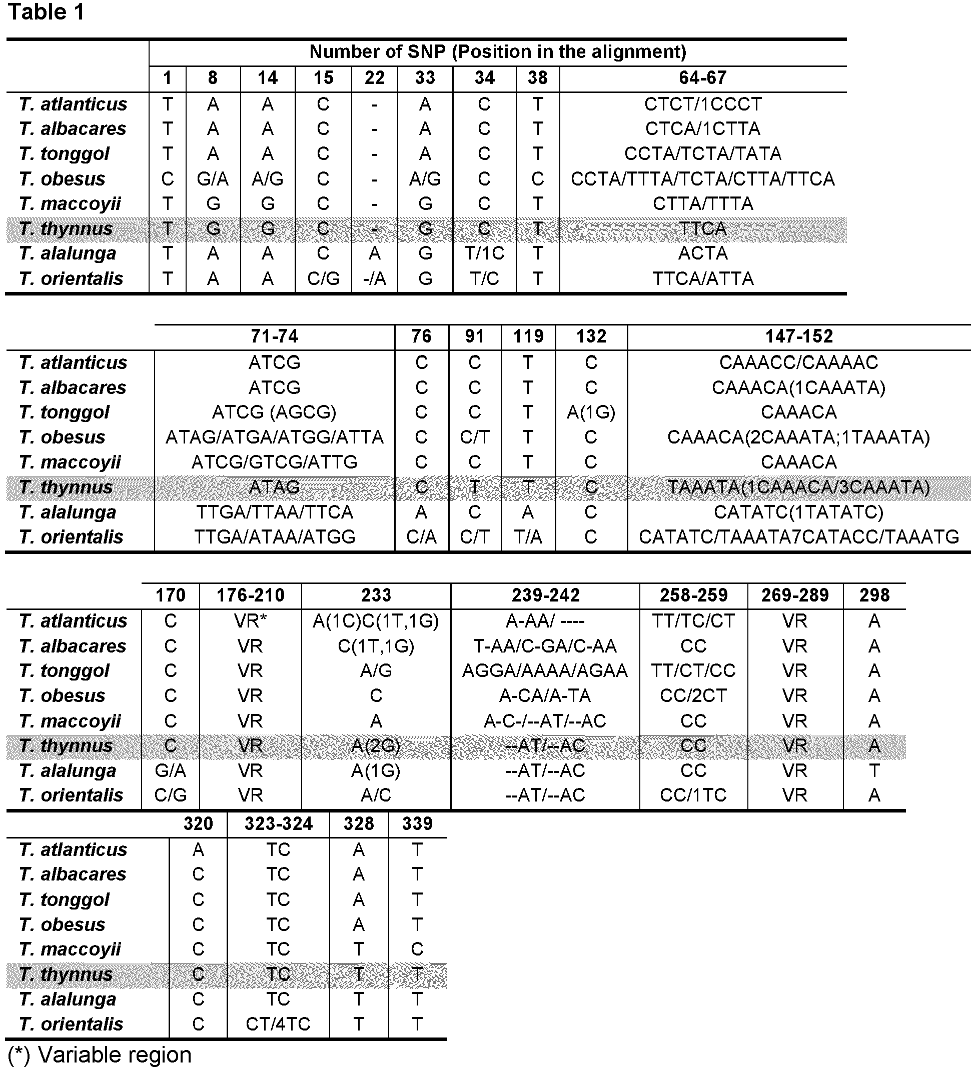

To study deeper these hypervariable regions and the differences among the *Thunnus* species, the alignment was filtered and cut. Consensus sequences for the target species and the most common substitutes *(T. albacares* and *T. obesus)* were included, combining the most common haplotypes in each species for that fragment. This second alignment is shown in **Figure 1**). Using this alignment and the consensus *T. thynnus* sequence as template, three potential sequences were designed to be used as primer Forward and another three as primer Reverse. The "PrimedRPA" software was used, and the primer options provided were adapted by the inventors in order to get higher specificity for *T. thynnus.* Table 2 shows the sequence of potential primers and RPA probes. The sequences in grey were subsequent analyzed.

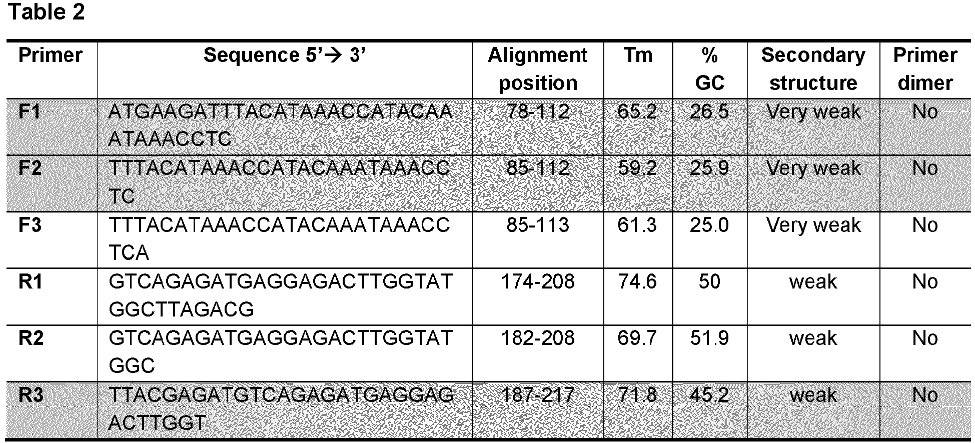

All possible combinations were studied on the alignment, *in silico* taking into account the characteristics of all the sequences (melting temperature, GC content, etc.) and also analyzing the specificity with the species of interest. From the six sequences selected, three were tested in the laboratory: the reverse primer R3 and the forward primers F1 and F2. Combinations of these primers were analyzed with all the species of the *Thunnus* genus, in order to check specificity to the target species *(T. thynnus)* and any cross amplification with the other species of the genus. 29.5 µL of buffer, 6.2 µL H₂O, 3.9 µL of primers F1 + R3 or F2 + R3 at 10 µM, and 2.5 µL of MgAc 14 mM) and were added to a tube of lyophilized RPA enzyme (TwistDX basic kit, TwisDX, Cambridge, UK). The mixture was divided in two new tubes (23 µL of reaction mix in each tube and 50 ng of template DNA was added to each tube. Isothermal PCR was performed for 20 minutes at 39 °C and maintenance at 4 °C, until the result was visualized on 2 % agarose gel stained with RedSafe solution (20,000×, Intron Biotechnology).

The expected amplicons were:
- F1+R3: 136 bp
- F2+R3: 129 bp

**Figure 2** shows the result of the amplification. Panel A shows the combination F1+R3 primer set, whereas panel B shows the combination F2+R3 primer set. As it may be appreciated only the combination, F2-R3 showed specific amplification only for *T. thynnus.* The other combination, F1-R3, showed amplification also for *T. maccoyii,* therefore it was considered non-specific.

The specific primers F2 (SEQ ID NO:1) and R3 (SEQ ID NO:2) were selected for the specific identification of *T. thynnus* in fish samples.

### Example 2. Design of the probes for amplification with nfo RPA and exo RPA

Once the best primer combination was determined in Example 1, the probes for using with the enzymes nfo RPA and exo RPA were designed using the *T. thynnus* consensus sequences as template. Three alternatives for each enzyme were designed and, after testing *in silico,* the probes Nfo2 and Exo2 were selected (Table 3). The sequences of theses probes are SEQ ID NO: 3 and SEQ ID NO:4 respectively. These sequences were analyzed, and the elements required for each enzyme were added (fluorophore, abasic site, 3'-spacers, quencher).

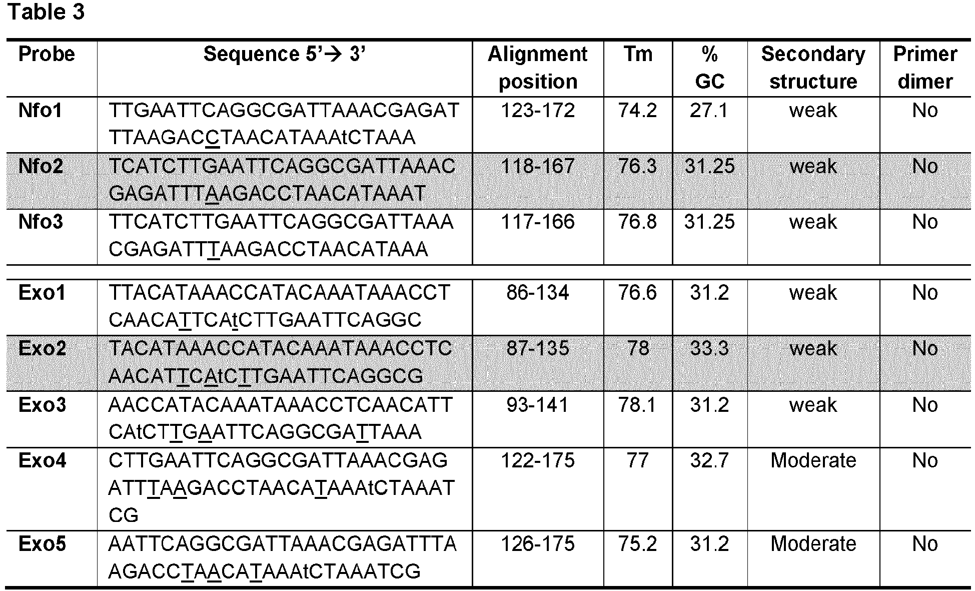

### Example 3. Identification of T. thynnus

In order to check that the selected probes were adequate in the identification method of *T*. *thynnus* with no cross-amplification with other tunids, and also compatible with rapid visualization by lateral flow assay (LFA). reactions with primers F2+R3 were performed.

DNA was extracted from the following species: *T. atlanticus, T. albacares, T. tonggol, T. obesus, T. maccoyii, T. thynnus, T. orientalis* and *T. alalunga.* The extraction was performed from a piece of muscle tissue, which was digested at 56 °C in a thermo shaker with 860 µL of lysis buffer (1% SDS (Sodium dodecyl sulfate), 150 mM NaCl, 2 mM EDTA (Ethylenediaminetetraacetic acid) and 10 mM Tris-HCI pH 8), 100 µL of 5 M guanidinium isothiocyanate and 40 µL of proteinase K (20 mg/mL). After 3 hours 40 µL of extra proteinase K was added and left overnight. DNA was isolated from the digested tissue with the Wizard DNA Clean-up System kit (Promega) following the manufacturer's protocol. The quantification of the double stranded DNA obtained was performed with a spectrophotometer

10.38 µL H₂O, 29.5 µL of buffer, 1.6 µL of each primer F2 and R3 10 µM, 0.6 µL of the probe Nfo2, and 2.32 µL of MgAc 14 mM and were added to a tube of lyophilized nfo RPA enzyme (TwistDX basic kit, TwisDX, Cambridge, UK). The mixture was divided in two new tubes (23 µL of reaction mix in each tube) and 50 ng of template DNA was added to each tube.

The reactions were incubated for 15 minutes in a Veriti thermal cycler (Applied Biosystems, Foster City, CA, USA) at 38 °C and RPA products were then diluted 1:50 with PBST running buffer. Afterwards, 10 µL of each diluted sample was transferred to the sample pad of a Hybridetect strip (Milenia Biotec GmbH, Gießen, Germany). Each strip was placed vertically with the sample pad submerged in 150 µL of PBST running buffer. After 4 min, the result was photographed and registered (**Figure 3**). The presence of two clear and distinguishable bands (upper control band and lower test band) on the strip indicated a positive result, while a negative result showed only the control band.

The samples assessed were: 1. Tthy12 *(T. thynnus),* 2. Tthy6 *(T. thynnus),* 3. Talb9 *(T. albacares),* 4. Tala11 *(T. alalunga),* 5. Tmac13 *(T. maccoyii*), 6. Tobe45 *(T. obesus),* 7. Tori17 *(T. orientalis),* 8. Tton1 *(T. tonggol*), 9. TatlTV28 *(T. atlanticus),* NEG: Negative control (no DNA).

The result shows the specificity of the primers/probe for identification of species *T. thynnus* from other species of the same genus.

Parallel results were obtained using the exo RPA enzyme. 12.38 µL H₂O, 29.5 µL of buffer, 1.6 µL of each primer F2 and R3 10 µM, 0.6 µL of the probe Exo2, and 2.32 µL of MgAc 14 mM, were added to a tube of lyophilized exo RPA enzyme (TwistDX basic kit, TwisDX, Cambridge, UK). Then, 50 ng of template DNA was added to each tube.

The reactions were incubated for 15 minutes in a 7500 fast real-time PCR System (Applied Biosystems, Foster City, CA, USA) at 38 °C and the fluorescence was measured each 1 minute. The results of fluorescence intensity were observed at real-time.

The samples assessed were: Tthy15 *(T. thynnus),* GAD1 *(T. thynnus),* BAL3 *(T. thynnus),* BAL1 *(T. thynnus),* Talb9 *(T. albacares),* Tobe45 *(T. obesus),* Tmac13 *(T. maccoyii*), Tala11 *(T. alalunga),* Tori27 *(T. orientalis),* Tatl18 *(T. atlanticus),* Tton17 *(T. tonggol*), NEG: Negative control (no DNA).

The result of the experiment is shown in **Figure 4****.** As it can be seen, there is a specific amplification only for the species *T. thynnus* and not for other species of the same genus (samples 1 to 4). Table 4 shows the amplification results of the RPA. Final Rn is the fluorescence registered at 15^{th} cycle, ΔRn is the interval of fluorescence detected and Ct is the cycle in which the amplification of that sample was registered. The first column shows the corresponding sample in Figure 4. The species marked in grey are those amplified by the exo RPA enzyme (all *T. thynnus).*

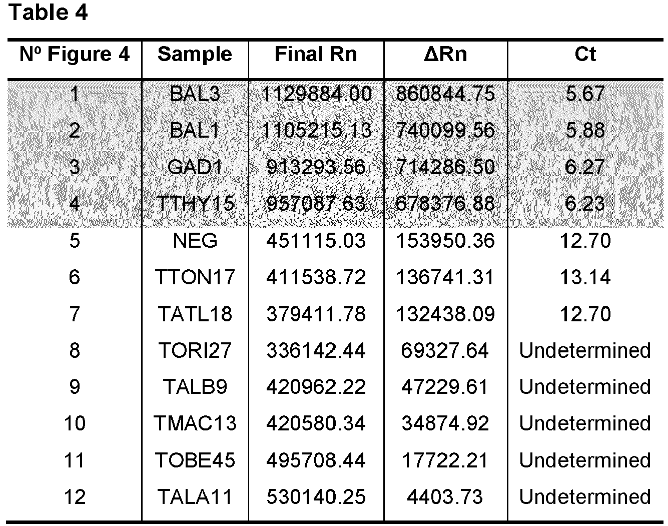

### SEQUENCE LISTING

SEQ ID NO:1: TTTACATAAACCATACAAATAAACCTC
SEQ ID NO:2: TTACGAGATGTCAGAGATGAGGAGACTTGGT
SEQ ID NO:3: TCATCTTGAATTCAGGCGATTAAACGAGATTTAAGACCTAACATAAAT
SEQ ID NO:4: TACATAAACCATACAAATAAACCTCAACATTCATCTTGAATTCAGGCG
SEQ ID NO:5: ATGAAGATTTACATAAACCATACAAATAAACCTC
SEQ ID NO:6: TTACATAAACCATACAAATAAACCTCA
SEQ ID NO:7: GTCAGAGATGAGGAGACTTGGTATGGCTTAGACG
SEQ ID NO:8: GTCAGAGATGAGGAGACTTGGTATGGC
SEQ ID NO:9: TTGAATTCAG GCGATTAAACGAGATTTAAGACCTAACATAAATCTAAA
SEQ ID NO:10: TTCATCTTGAATTCAGGCGATTAAACGAGATTTAAGACCTAACATAAA
SEQ ID NO:11: TTACATAAACCATACAAATAAACCTCAACATTCATCTTGAATTCAGGC
SEQ ID NO:12: AACCATACAAATAAACCTCAACATTCATCTTGAATTCAGGCGATTAAA

SEQ ID NO:13: CTTGAATTCAGGCGATTAAACGAGATTTAA GACCTAACATAAATCTAAAT
CG

SEQ ID NO: 14: AATTCAGGCGATTAAACGAGATTTAAGACCTAACATAAATCTAAATCG

## Claims

1. A process for identification of bluefish tuna *(Thunnus thynnus)* from a fish sample comprising:
a) DNA isolation from the sample;
b) adding to the isolated DNA of step a) a forward primer of sequence SEQ ID NO:1 and a reverse primer of SEQ ID NO:2;
c) adding an enzyme;
d) incubating the mixture of step c) between 10 and 30 minutes at 25 - 42 °C; and
e) visualization of the result,
wherein the enzyme is selected from a recombinase (RPA), a DNA helicase (HAD) or a strand-displacement enzyme (SDA).

2. The process of claim 1, wherein the enzyme is a recombinase (RPA) selected from nfo RPA, exo RPA and fpg RPA and a probe is added.

3. The process of claim 2, wherein the enzyme is nfo RPA.

4. The process of claim 3, wherein the reverse primer SEQ ID NO:2 is labeled with a fluorophore at the 5'-end.

5. The process of claim 4, wherein the label is biotin.

6. The process of claims 3 to 5, wherein the probe is defined by SEQ ID NO:3.

7. The process of claim 6, wherein the probe is labeled with a flurorophore in 5' position, a blocker in 3' and the sequence comprises an abasic site between positions 30 and 35 from 5' of SEQ ID NO:3.

8. The process of claim 7, wherein the fluorophore is FAM, the blocker is a C3-space and the abasic site is thetrahydrofurane (THF) at position 33 of SEQ ID NO:3.

9. The process of claim 2, wherein the enzyme is exo RPA.

10. The process of claim 9, wherein the probe is defined by SEQ ID NO:4.

11. The process of claim 10, wherein the probe is labeled with a flurorophore, a blocker in 3', a quencher and an abasic site between positions 30 and 35 from 5' of SEQ ID NO:3.

12. The process of claim 11, wherein the fluorophore is FAM located at position 31 of SEQ ID NO:4, the blocker is a C3-space, the quencher is BHQ1 at position 36 of SEQ ID NO:4 and the abasic site is thetrahydrofurane (THF) at position 33 of SEQ ID NO:4, from 5'.

13. the process of claims 1 to 12, wherein the mixture is incubated in step d) for 15 minutes at 38 °C.

14. The process of claims 1 to 13, wherein the detection method is lateral flow strip detection when the enzyme is RPA nfo, RPA fpg or SDA.


SEQUENCE LISTING

<110> CONSEJO SUPERIOR DE INVESTIGACIONES CIENTÍFICAS
      INL - International Iberian Nanotechnology Laboratory

<120> METHOD FOR THE IDENTIFICATION AND RAPID DETECTION OF BLUEFIN TUNA

<130> 2020/36587

<160> 14

<170> BiSSAP 1.3.6

<210> 1
<211> 27
<212> DNA
<213> Artificial Sequence

<220>
<223> Primer F2

<400> 1
tttacataaa ccatacaaat aaacctc                                          27

<210> 2
<211> 31
<212> DNA
<213> Artificial Sequence

<220>
<223> Primer R3

<400> 2
ttacgagatg tcagagatga ggagacttgg t                                     31

<210> 3
<211> 48
<212> DNA
<213> Artificial Sequence

<220>
<223> Probe nfo RPA 2

<400> 3
tcatcttgaa ttcaggcgat taaacgagat ttaagaccta acataaat                   48

<210> 4
<211> 48
<212> DNA
<213> Artificial Sequence

<220>
<223> Probe exo RPA 2

<400> 4
tacataaacc atacaaataa acctcaacat tcatcttgaa ttcaggcg                   48

<210> 5
<211> 34
<212> DNA
<213> Artificial Sequence

<220>
<223> Primer F1

<400> 5
atgaagattt acataaacca tacaaataaa cctc                                    34

<210> 6
<211> 28
<212> DNA
<213> Artificial Sequence

<220>
<223> Primer F3

<400> 6
tttacataaa ccatacaaat aaacctca                                           28

<210> 7
<211> 34
<212> DNA
<213> Artificial Sequence

<220>
<223> Primer R1

<400> 7
gtcagagatg aggagacttg gtatggctta gacg                                    34

<210> 8
<211> 27
<212> DNA
<213> Artificial Sequence

<220>
<223> Primer R2

<400> 8
gtcagagatg aggagacttg gtatggc                                            27

<210> 9
<211> 48
<212> DNA
<213> Artificial Sequence

<220>
<223> Probe nfo RPA 1

<400> 9
ttgaattcag gcgattaaac gagatttaag acctaacata aatctaaa                48

<210> 10
<211> 48
<212> DNA
<213> Artificial Sequence

<220>
<223> Probe nfo RPA 3

<400> 10
ttcatcttga attcaggcga ttaaacgaga tttaagacct aacataaa                48

<210> 11
<211> 48
<212> DNA
<213> Artificial Sequence

<220>
<223> Probe exo RPA 1

<400> 11
ttacataaac catacaaata aacctcaaca ttcatcttga attcaggc                48

<210> 12
<211> 48
<212> DNA
<213> Artificial Sequence

<220>
<223> Probe exo RPA 3

<400> 12
aaccatacaa ataaacctca acattcatct tgaattcagg cgattaaa                48

<210> 13
<211> 52
<212> DNA
<213> Artificial Sequence

<220>
<223> Probe exo RPA 4

<400> 13
cttgaattca ggcgattaaa cgagatttaa gacctaacat aaatctaaat cg           52

<210> 14
<211> 48
<212> DNA
<213> Artificial Sequence

<220>

<223> Probe exo RPA 5

<400> 14
aattcaggcg attaaacgag atttaagacc taacataaat ctaaatcg                    48